# EUROPEAN PATENT SPECIFICATION

(11) **EP 0 739 195 B1**
(45) Date of publication and mention of the grant of the patent: **13.09.2000**
(21) Application number: 95904310.0
(22) Date of filing: 08.12.1994
(51) Int. Cl.: A61K 7/42, A61K 7/48

(54) **SKIN TANNING COMPOSITIONS**
Hautbräunungsmittel
COMPOSITIONS AUTOBRONZANTES

(30) Priority: 09.12.1993 US 164519
(43) Date of publication of application: 30.10.1996
(73) Proprietor: ESTEE LAUDER INC., New York New York 10153 (US)
(72) Inventor: LENTINI, Peter, Glen Oaks, NY 11004 (US); MARENUS, Kenneth, Dix Hills, NY 11746 (US); MUIZZUDDIN, Neelam, Farmingdale, NY 11735 (US); PELLE, Edward, Valley Stream, NY 11580 (US); PUNTO, Louis, St. Petersburg, FL 33716 (US)
(74) Representative: Pochart, François
(86) International application number: PCT/US94/14190
(87) International publication number: WO 95/15742

(56) References cited:
- EP-A- 0 547 864
- WO-A-92/00303
- FR-A- 2 680 684
- GB-A- 928 266
- US-A- 3 177 120

## Description

The invention generally relates to the field of cosmetic compositions. More particularly, the invention relates to compositions and preparations which are applicable to the human skin for imparting thereto a simulated tan such as would be normally acquired by exposure to the sun.

### BACKGROUND OF THE INVENTION

Many individuals have a skin complexion which does not tan readily on exposure to sun light. Others achieve a tan only with great discomfort and possibly adverse effects to the skin due to exposure to the suns rays. Yet attainment of a tan by many individuals is highly desired for cosmetic and other reasons, especially if this can be accomplished without the usual exposure to the sun.

In other instances, individuals who tan with difficulty may desire to extend the life of a naturally acquired tan without re-exposure to the sun. Also, a skin tan may be desired when weather conditions do not permit the usual exposure to the sun in order the acquire a tan.

Acquisition of a natural tan by exposure to the sun, however, may be almost impossible for those very light skinned persons who tend to burn rather than tan. In addition, the deleterious effects of excessive exposure to sunlight are becoming more generally recognized.

One of the most common methods for artificially inducing a suntan is to subject the body to the rays of an ultraviolet ray lamp. While this induces a tan, it has many of the same disadvantages as tanning by the sun since many of the deleterious effects of sunlight are due to its ultraviolet radiation component. For instance, the increasing incidence of skin cancer has been attributed to increased exposure to ultraviolet radiation from the sun.

It is known in the art to induce an artificial tan by applying dihydroxyacetone ("DHA") to the human skin by a Suitable vehicle or base. Darkening of the human skin occurs within about 2-24 hours after applying a suitable composition containing DHA as the active agent. See U.S. Pat. No. 2, 949, 403.

EP0547 864 discloses a cosmetic method for imparting a natural-appearing tan to skin using a self-tanner cosmetic product which is a multi-compartment dispenser wherein a first and second substance are stored in separate compartments thereof, at least one of the substances including from 0.1 to 60 % of a silicone ; the first substance comprising from 0.1 to 40 % of a C3-C24 alpha-hydroxy aldehyde, and an effective amount of a pharmaceutically acceptable vehicle for supporting the alpha-hydroxy aldehyde ; and the second substance comprising from 0.01 to 25 % of at least one amino acid, and an effective amount of a pharmaceutically acceptable vehicle for delivering at least one amino acid.

While DHA has been widely commercialized as a skin tanning agent, formulations containing DNA suffer from a number of deficiencies. A particular disadvantage is the time required to produce the desired tanning effect. A further disadvantage is that the tan imparted by DHA readily is washed off the skin. A need therefore exists for skin tanning formulations which more quickly produce the desired tanning effect. A further need exists for skin tanning formulations which are less readily be removed from the skin.

### SUMMARY OF THE INVENTION

The disclosed invention is directed to methods and compositions for improving the rate, extent and lifetime of an artificially induced tan of the human skin.

The present invention thus covers a cosmetic composition applicable to the human skin for imparting an artificial tan thereto comprising,
(1) a first component comprising about 1-20 percent DHA; and
(2) a second component comprising at least about 2 percent of an amino-containing component selected from the group consisting of amino-substituted silicones and polyaziridines, wherein the second component is available for reaction with said first component to increase the rate and degree of tan imparted to the human skin.

According to a specific embodiment said first component is an oil in water emulsion containing about 5-8 % DHA.

According to another embodiment of the composition of the invention, said first component comprises at least one of either a water-alcohol solution, an aqueous, oil-free, alcohol-free spray-on emulsion, or an oil in water emulsion ; and according to this specific embodiment said DHA can represent about 5-8 % of said first component.

Furthermore the present invention provides a method of imparting an artificially induced tan to the human skin comprising,
(1) treating the skin with a first composition having at least about 2 % of an amino containing component selected from the group consisting of amino-substituted silicones and polyaziridines therein, and
(2) applying to the skin a second composition having about 1-20 % DHA therein.
According to a specific embodiment of the method of the invention, said first composition is at least one of either a water-alcohol solution or an oil-in-water emulsion and the DHA can represent about 1 % of said second composition or can represent about 5-8 % of said second composition.

According to another embodiment said first composition includes polyethyleneimine and said second composition is a water-in-oil emulsion containing DHA.

According to another embodiment said first composition comprises an amino substituted silicone of formula (I) 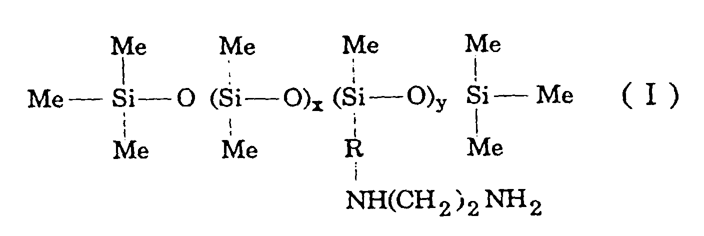 Wherein :
x = 48-148,
Y = 3-15, and
R is a divalent alkylene radical of 3-6 carbon atoms and said second composition is a water-in-oil emulsion containing DHA.

The other specific embodiments of the method and composition of the present invention are defined in the dependent claims.

In accordance with the invention, a composition applicable to the human skin and which is suitable for imparting a tan thereto is provided. Preferably, the composition is in the form of a spray or an emulsion. The composition includes a component having DHA, and an amino containing component selected from the group consisting of polyaziridines and amino substituted silicones of formula I: 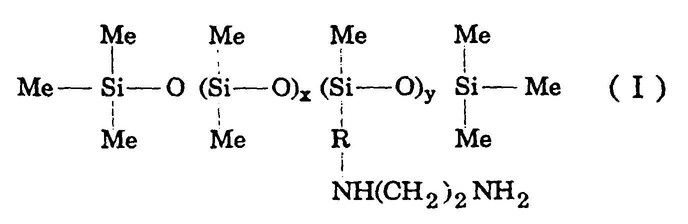 where:
x = 48-148
y = 3-15 and
R is a divalent alkylene radical of 3-6 carbon atoms
The amino substituted silicones of Formula (I) are available from Dow Corning under the name DCX28124. The amino containing component is capable of reacting with the first component to increase the rate and degree of tan imparted to the human skin.

The invention also is directed to a method for increasing the rate of artificially tanning the human skin and retention of that tan by applying to the skin a composition containing amino groups prior or simultaneously with application of DHA. The amino groups are provided in the form of amino substituted silicones of the above formula (I) or polyaziridines such as polyethyleneimine and the like.

In yet another aspect of the invention, a cosmetic preparation applicable to the human skin for imparting a tan within fifteen minutes after application thereto is provided.

### DETAILED DESCRIPTION OF THE INVENTION

Generally, and in accordance with the invention, the rate and degree of artificial tanning imparted to the human skin by DHA, as well as the lifetime of that tan, surprisingly can be increased by treating the skin with selected amino containing compounds prior to, subsequent to, or simultaneously with applying DHA containing compositions.

The DHA composition in various vehicles can be applied to the skin subsequent to or simultaneously with applying the amino containing component. The DHA composition can be applied in the form of a spray of DHA in a hydroalcohol solution such as water-ethanol; an aqueous, oil-free, alcohol-free spray-on emulsion, or preferably as a cream in the form of DHA in an emulsion such as oil in water. Useful oils include silicone oils, mineral oils, as well as naturally derived or synthetic plant or animal oils. Examples of useful oils include but are not limited to dimethicone, sweet almond oil, rice oil, squalene, shark liver oil, mink oil, liquid oil esters, liquid oil ethers, diisopropyl adipate, and polypropylene glycol methyl ether.

The percentages of DHA in the vehicle can vary over a wide range. When employed in a spray, the amount of DHA typically is 1-20%, preferably 5-8%, based on the liquid vehicle of the spray. When employed as a cream of an oil in water emulsion, DHA typically is 1-20%, preferably 5-8%, based on the emulsion.

In an embodiment of the invention as illustrated in examples 1-2 polyethyleneimine is applied to the skin prior to application of DHA. DHA is applied as a mixture of 5% DHA, 21% water, and 32% magnesium alumino-silicate. The polyazaridines employed have the formula (II): 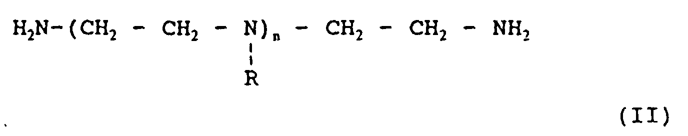 wherein:
R = H or CH₂ - CH₂ - NH - CH₂ - NH₂; and n is an integer chosen such that the molecular weight of the polyethyleneimine is between 500 and 2,000,000, preferably between 400,000 and 800,000, most preferably between 500,000 and 700,000. These polyethyleneimines are commercially available from a number of suppliers. For example, polyethyleneimines are sold by BASF Corporation (Parsippany, NJ), under the tradename Polymin®.

Polyethyleneimine is a polyaziridine. Polyaziridines other than polyethyleneimine can be employed. Examples of useful polyaziridines include but are not limited to polyethyleneimine and the like. The amino containing polymers may be diluted in water-alcohol solutions such as water-ethanol to concentrations as low as 2%. The polymers also may be applied in neat form.

In examples 1-2 areas of skin are treated with solutions of polyethyleneimine and absorbed for 50-60 minutes. Then, a thin layer of the composition having 5% DHA as described above is applied to the treated skin. For comparison, in Example 3 the same 5% DHA composition is applied to an untreated portion of skin. Skin tanning in terms of increase in skin color is measured with the Muiolta chromameter model No CR200 before treatment to obtain baseline data, and after 5 hours, 24 hours and 5 days after treatment. The results are given in Table 1.

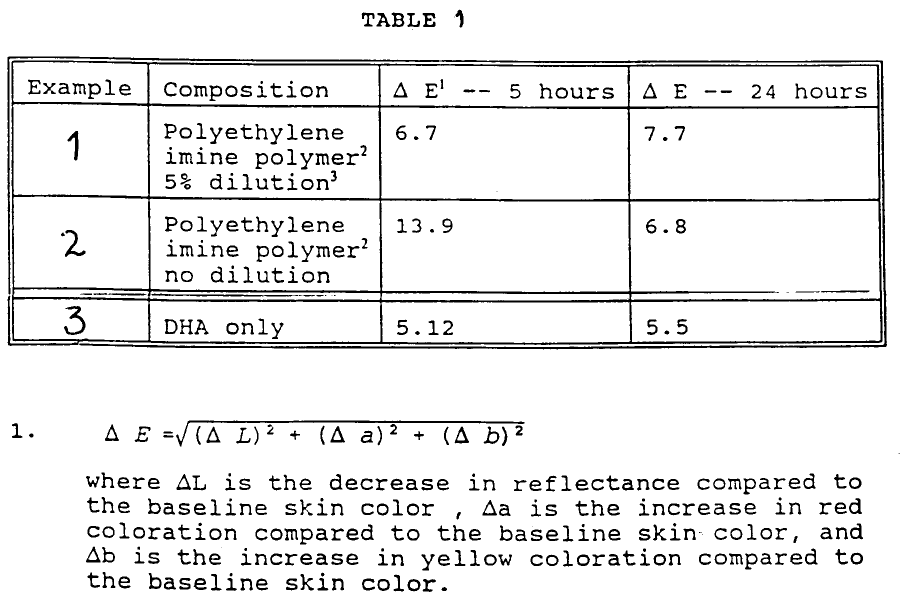

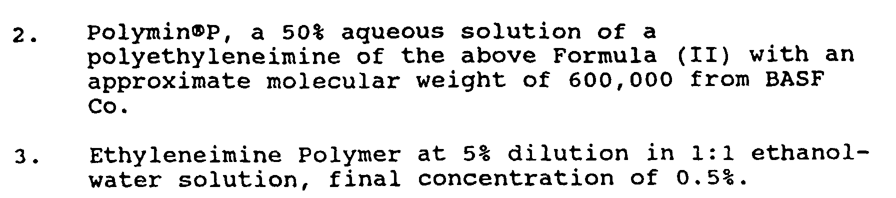

In examples 1-2, the amino containing material (polyethyleneimine) is applied to the skin prior to applying the DHA containing composition. In examples 4-5, the amino containing material is applied to the skin as a mixture of the amino containing material and a hydroalcoholic solution (EL Self Protection Tonic, available from Estée Lauder, Inc.), prior to applying the DHA containing material. The effects of including the amino containing material in the EL Self Protection Tonic are shown in Table 2 below. The tanning effect is determined in the manner given above. For comparison, in Examples 6 and 7 the tanning effects of the EL Self Action Tanning Cream (Medium) and DHA containing compositions on untreated skin are measured.

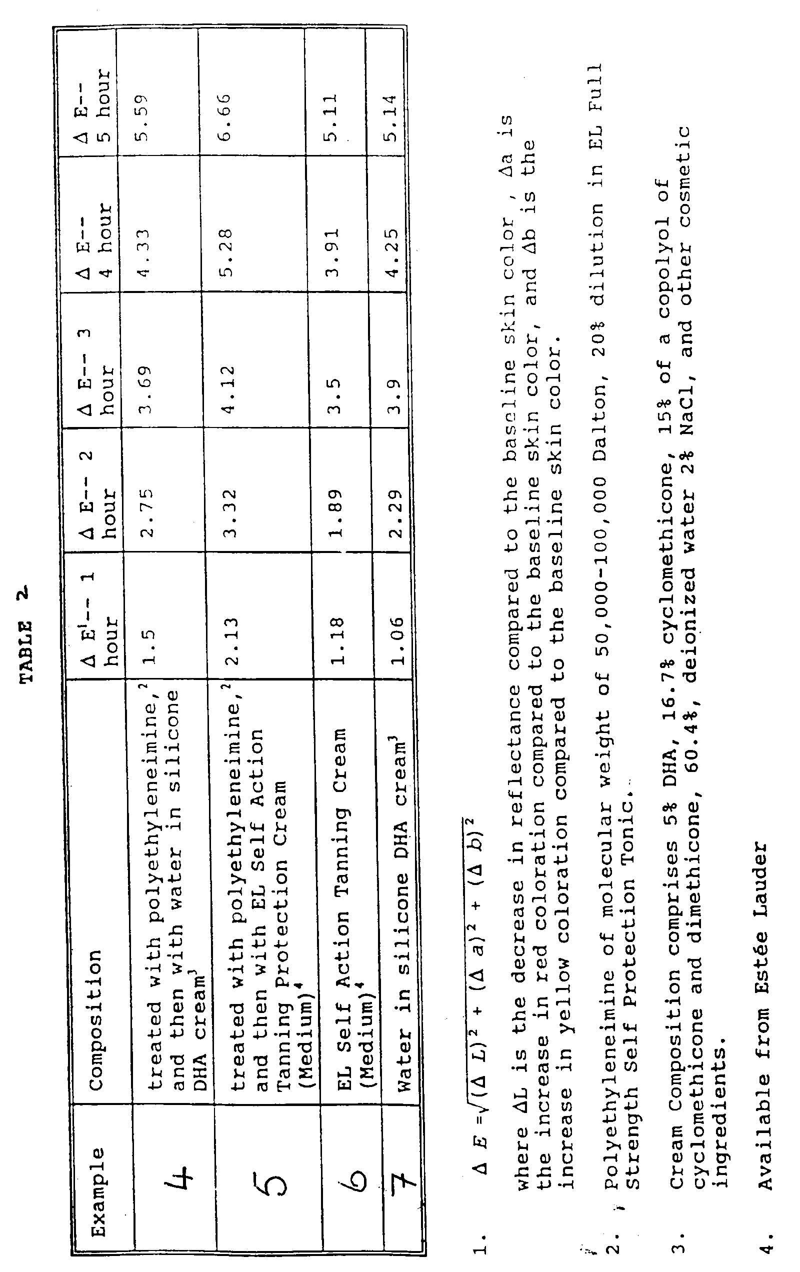

### Examples 8-14

The compositions of Examples 8-14 further illustrate the invention. The compositions of examples 8-14 are made by dissolving each ingredient in order of appearance. These compositions require no special processing other than a prop mixer and an appropriately sized mixing vessel.

**TABLE 3**

| Exam. No. /Constituent | 8 | 9 | 10 | 11 | 12 | 13 | 14 |
|---|---|---|---|---|---|---|---|
| Deionized water | 79.00 | 78.00 | 79.00 | 78.00 | 0.00 | 0.00 | 0.00 |
| Polymin P | 4.00 | 0.00 | 0.00 | 0.00 | 0.00 | 0.00 | 0.00 |
| 1,3-B.² Glycol | 10.00 | 10.00 | 10.00 | 10.00 | 0.00 | 0.00 | 0.00 |
| Fragrance | 0.50 | 0.50 | 0.50 | 0.50 | Remainder | Remainder | Remainder |
| Tween 20, NF³ | 0.50 | 0.50 | 0.50 | 0.50 | 0.00 | 0.00 | 0.00 |
| Liponic EG-1⁴ | 5.00 | 5.00 | 5.00 | 5.00 | 0.00 | 0.00 | 0.00 |
| Ucon 50HB660⁵ | 1.00 | 1.00 | 1.00 | 1.00 | 1.00 | 0.00 | 0.00 |
| Polymin G-20 | 0.00 | 5.00 | 0.00 | 0.00 | 0.00 | 0.00 | 0.00 |
| Polymin FG' | 0.00 | 0.00 | 0.00 | 5.00 | 0.00 | 0.00 | 0.00 |
| Polymin G-35' | 0.00 | 0.00 | 4.00 | 0.00 | 0.00 | 0.00 | 0.00 |
| Polymin Waterfree' | 0.00 | 0.00 | 0.00 | 0.00 | 2.00 | 0.00 | 2.00 |
| DC X2-8124 | 0.00 | 0.00 | 0.00 | 0.00 | 0.00 | 2.00 | 2.00 |
| Ceraphyl ICA⁶ | 0.00 | 0.00 | 0.00 | 0.00 | 10.00 | 10.00 | 10.00 |
| DC Q2-1401⁷ | 0.00 | 0.00 | 0.00 | 0.00 | 1.00 | 1.00 | 1.00 |
| DC 344⁷ | 0.00 | 0.00 | 0.00 | 0.00 | 80.00 | 80.00 | 78.00 |
| DC 345⁷ | 0.00 | 0.00 | 0.00 | 0.00 | 5.00 | 5.00 | 5.00 |
| DC 200/20cts⁷ | 0.00 | 0.00 | 0.00 | 0.00 | 2.00 | 2.00 | 2.00 |

| | | | | | | | |
|---|---|---|---|---|---|---|---|
| 1. Polymin P, G-20, FG, G-35, Waterfree; available from BASF | | | | | | | |
| 2. 1,3-B. Glycol from Robeco Chemicals | | | | | | | |
| 3. Tween 20, NF from ICI - Polysorbate 20 | | | | | | | |
| 4. Liponic EG1 from Lipo Chemicals - Glysereth 23 | | | | | | | |
| 5. UCON 50-HB-660 from Amerchol - PPG-12-Butech-16 | | | | | | | |
| 6. Ceraphyl ICA is available from Van Dyk Isocetyl Alcohol | | | | | | | |
| 7. DC X2-8124, 1401, 344, 345, 200/20cts from Dow Corning | | | | | | | |

In examples 1-2, 4, and 5, the amino containing material is applied prior to treating with DHA. However, in an alternative embodiment, and as illustrated in Example 15 below, the amino containing material is applied simultaneously with DHA.

### Examples 15-16

Duo phase compositions formed of an amino containing polymer such as polyethyleneimine with DHA containing compositions are shown in example 15. These duo phase compositions provide DHA in the first phase, and polyethyleneimine in the second phase. The first phase that contains DHA may include hydroalcohol sprays, aqueous, oil-free, alcohol-free spray-on emulsions, or oil in water emulsions. The relative percentages of the first and second phases in the duo phase composition may vary over a wide range. The amount of DHA in the first phase may vary from 1 to 20%, preferably 4 to 8%, of the duo phase composition. The second phase may include a gel that contains 10-100% polyathyleneimine.

The first and second phases are applied simultaneously to the skin. The surprising tanning effects attributable to use of the duo phase compositions is given below in Table 4. For comparison, and as shown in Example 16 the tanning effects due to commercially available, EL Self Action Tanning Medium containing DHA are provided.

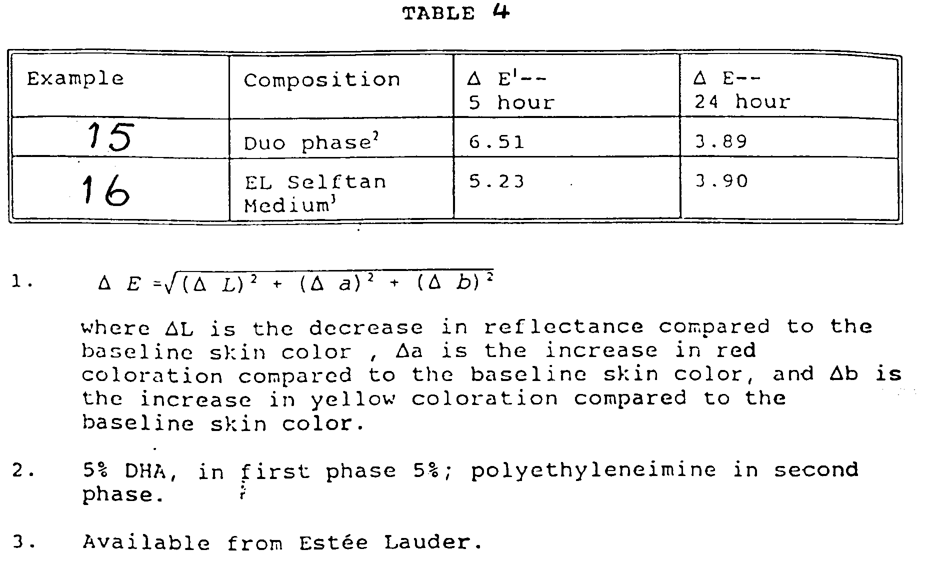

### Examples 17-22

Table 5 illustrates the effect of the amino containing compounds on the onset and intensity of the tanning of human skin by treating the skin with a DHA composition. Compositions containing 3.75% and 5.00% of DC X2-8124 were utilized as the second component and compositions containing 5.0% and 6.5% DHA were the first component of the presently claimed invention.

5 groups of panelists were treated as follows: Example 17 treated on one arm with AS 2295/1 (3.75% DC X2-1824) followed by Supertan Medium (5% DHA) and only Supertan Medium (5% DHA) on the other arm; Example 18 treated on one arm with AS 2295/2 (5% DC X2-1824) followed by Supertan Medium (5% DHA) and only Supertan Medium (5% DHA) on the other arm; Example 19 treated on one arm with AS 2295/1 (3.75% DC X2-1824) followed by Supertan Dark (6.5% DHA) and only Supertan Dark (6.5%) on the other arm; Example 20 treated on one arm with AS 2295/2 (5% DC X2-1824) followed by Supertan Dark (6.5% DHA) and only Supertan Dark (6.5% DHA) on the other arm; Examples 21 and 22 treated one arm only with Supertan Dark (6.5% DHA)(Ex. 21) and only Supertan Medium. (5% DHA) on the other (Ex. 22)

In the examples described above, 600 µl of the second component was applied and allowed to dry. Subsequently, 800 µl of the first component was applied to the skin and rubbed in until absorbed. Likewise, 800 µl of the first component was applied to skin that did not receive pretreatment. Color measurements were obtained with a chromameter before treatment (baseline) and then after 10 minutes, 20 minutes, 30 minutes, 1 hour, 2 hours, 3 hours, 5 hours and 24 hours.

Clearly, as presented in Table 5, the application of the Primer Toner containing the DC X2-8124 prior to applying the DHA-containing compositions results in a substantially faster rate and degree of tan as compared to treatment with a DHA-containing compound alone.

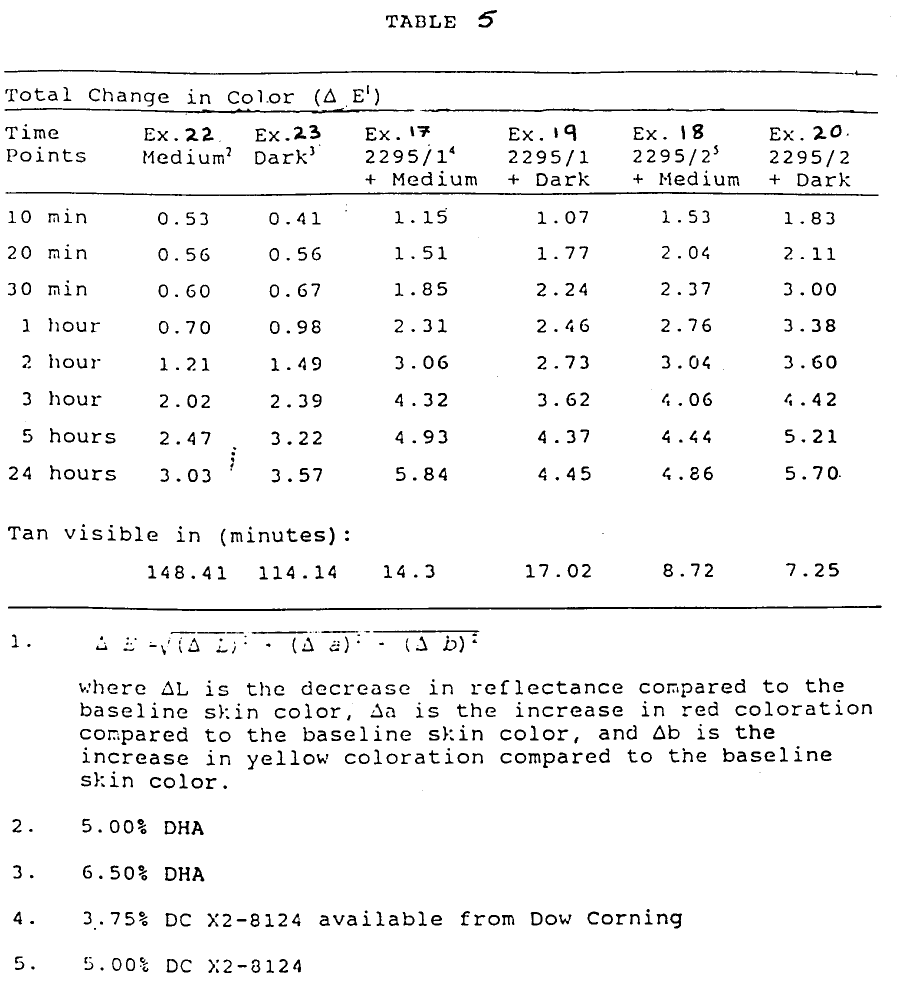

### Examples 23-32

Additionally, Tables 6 and 7 further illustrate the tan enhancing effects of the compositions of the presently claimed invention. The effect of AS 2293/10 (5% DC X2-8124) was tested with a variety of E.L Selftan products. The following Selftan products (with corresponding DHA% levels) were tested: Selftan Cream Very Dark (6.5% DHA), Selftan Cream Medium (5% DHA), Supertan Cream For the Face Dark (6.5% DHA), Selftan cream light (2.5% DHA), Supertan for the Face Medium (6% DHA) Selftan Cream Dark (6.5% DHA), Self Action Spray Medium (5% DHA), Self Action Spray Dark (7% DHA,) Supertan Dark (6.5% DHA), and Supertan Medium (5% DHA).

A group of 6 or 7 test subjects was selected for each Selftan Product. The AS 2293/10 ( 5 % DC X2-8124) was applied to one arm of the test subjects and allowed to dry for 2-4 minutes. 600 µl of a Selftan composition was then rubbed in on each arm until absorbed. Skin tanning, in terms of increase in skin color was measured with a chromameter before treatment (baseline), after 10 minutes, 20 minutes, 30 minutes, 1 hour, 2 hours, and 5 hours.

As shown in Tables 6 and 7, the skin pretreated with the Primer Toner exhibited a considerably higher rate and degree of tan as compared to skin treated solely with the DHA-containing Selftan product. For virtually all of the Selftan products, there was a striking difference in the total change in color, as well as the time that it took for the onset of Selftan.

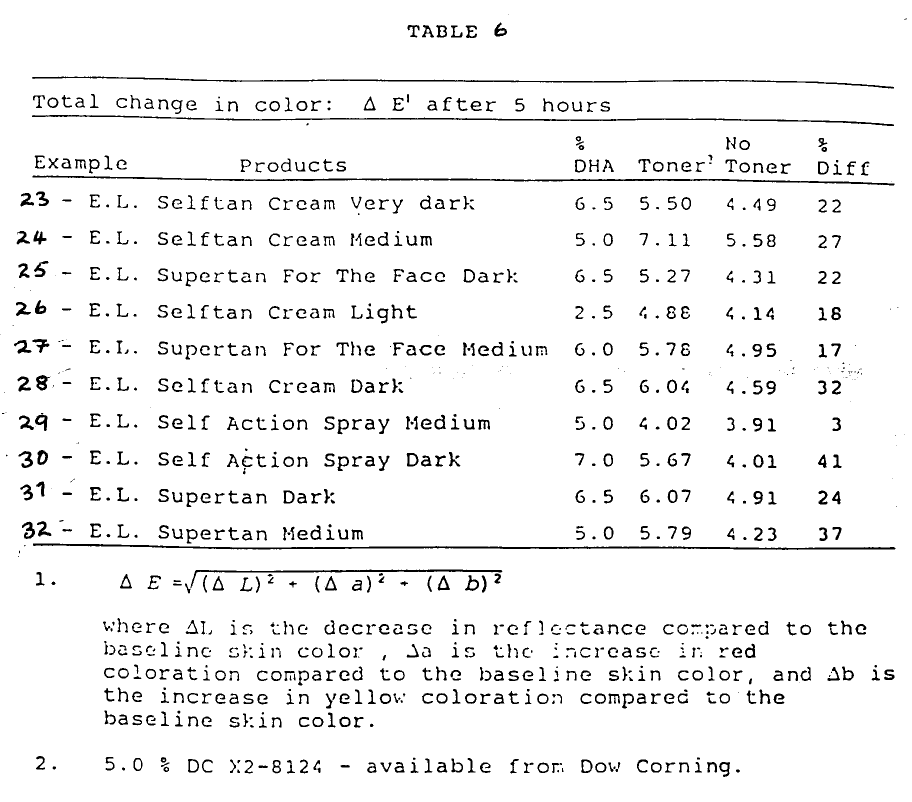

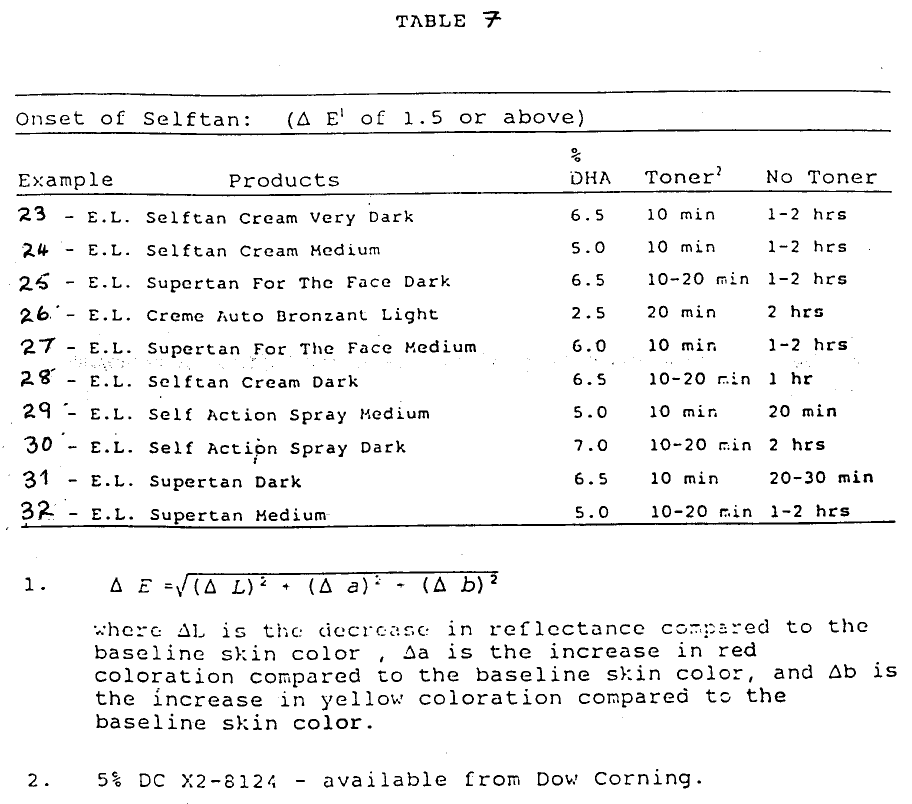

## Claims

1. A cosmetic composition applicable to the human skin for imparting an artificial tan thereto comprising,
a first component comprising about 1-20 percent DHA; and
a second component comprising at least about 2 percent of an amino-containing component selected from the group consisting of amino-substituted silicones and polyaziridines, wherein the second component is available for reaction with said first component to increase the rate and degree of tan imparted to the human skin.

2. The composition of claim 1 wherein said first component comprises at least one of either a water-alcohol solution, an aqueous, oil-free, alcohol-free spray-on emulsion, or an oil in water emulsion.

3. The composition of claim- 2 wherein said water-alcohol solution comprises water and ethanol.

4. The composition of claim 2 wherein said DHA is about 5-8% of said first component.

5. The composition of claim 1 wherein said first component is an oil in water emulsion containing about 5-8% DHA.

6. The composition of claim 1 wherein said polyaziridine is polyethyleneimine.

7. The composition of claim 1 wherein said first component is an oil in water emulsion containing DHA and said second component is polyethyleneimine.

8. The composition of claim 1 wherein said first component is an oil in water emulsion containing DHA and said amino containing component is an amino substituted silicone.

9. The composition of claim 8 wherein said amino substituted silicone is a compound of Formula (I) 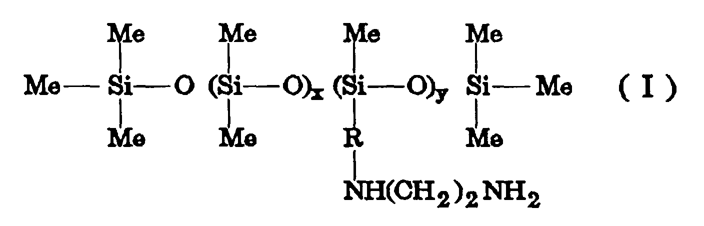 wherein:
x = 48-148,
y = 3-15, and
R is a divalent alkylene radical of 3-6 carbon atoms.

10. A method of imparting an artificially induced tan to the human skin comprising,
treating the skin with a first composition having at least about 2% of an amino containing component selected from the group consisting of amino-substituted silicones and polyaziridines therein, and
applying to the skin a second composition having about 1-20% DHA therein.

11. The method of claim 10 wherein said first composition is at least one of either a water-alcohol solution or an oil-in-water emulsion.

12. The method of claim 11 wherein said water-alcohol solution is water ethanol.

13. The method of claim 11 wherein DHA is at least about 1% of said second composition.

14. The method of claim 13 wherein said DHA is about 5-8% of said second composition.

15. The method of claim 10 wherein said second composition is an oil in water emulsion containing about 5-8% DHA.

16. The method of claim 10 wherein said polyaziridine is polyethyleneimine.

17. The method of claim 10 wherein said first composition includes polyethyleneimine and said second composition is a water-in-oil emulsion containing DHA.

18. The method of claim 10 wherein said first composition comprises an amino substituted silicone of Formula (I) 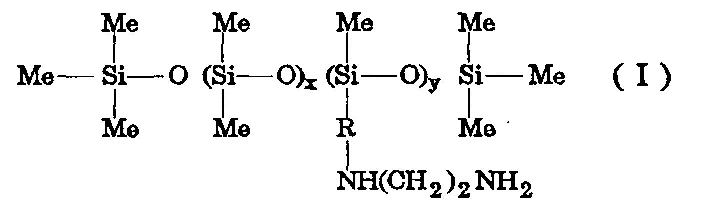 wherein:
x = 48-148,
y = 3-15, and
R is a divalent alkylene radical of 3-6 carbon atoms;
and said second composition is a water-in-oil emulsion containing DHA.

19. The method of claim 10 wherein said treating with said first composition and said applying of said DHA containing composition is performed substantially simultaneously.

20. The method of claim 10 wherein said first and second compositions are applied simultaneously.

21. The method of claim 10 wherein said first and second compositions are in a duo phase composition.

22. A method of imparting an artificially induced tan to the human skin according to claim 10 comprising:
(a) treating the skin with a first composition comprising at least about 2 percent of an amino containing component selected from the group consisting of an amino substituted silicone or at least one polyaziridine; and
(b) applying to the skin a second composition having about 1-20 percent DHA therein.

23. The method of claim 22 wherein said first and second compositions are applied simultaneously.

24. The method of claim 22 wherein said first and second compositions are in a duo phase composition.

## Patentansprüche

1. Kosmetische Zusammensetzung, die auf die menschliche Haut auftragbar ist, um ihr eine künstliche Sonnenbräune zu verleihen, die umfaßt,
einen ersten Bestandteil, der etwa 1-20% DHA umfaßt; und
einen zweiten Bestandteil, der wenigstens etwa 2% eines amino-enthaltenden Bestandteils umfaßt, der ausgewählt ist aus der Gruppe, die besteht aus aminosubstituierten Siliconen und Polyaziridinen, wobei der zweite Bestandteil für die Reaktion mit dem ersten Bestandteil zur Verfügung steht, um die Geschwindigkeit und den Grad der Sonnenbräune, die der menschlichen Haut verliehen wird, zu erhöhen.

2. Zusammensetzung nach Anspruch 1, wobei der erste Bestandteil wenigstens eines von einer Wasser-Alkohollösung, einer wäßrigen, öl-freien, alkohol-freien, aufsprühbaren Emulsion oder einer Öl-in-Wasser-Emulsion umfaßt.

3. Zusammensetzung nach Anspruch 2, wobei die Wasser-Alkohollösung Wasser und Ethanol umfaßt.

4. Zusammensetzung nach Anspruch 2, wobei DHA etwa 5-8% des ersten Bestandteils ausmacht.

5. Zusammensetzung nach Anspruch 1, wobei der erste Bestandteil eine Öl-in-Wasser-Emulsion ist, die etwa 5-8% DHA enthält.

6. Zusammensetzung nach Anspruch 1, wobei das Polyaziridin Polyethylenimin ist.

7. Zusammensetzung nach Anspruch 1, wobei der erste Bestandteil eine Öl-in-Wasser-Emulsion ist, die DHA enthält, und der zweite Bestandteil Polyethylenimin ist.

8. Zusammensetzung nach Anspruch 1, wobei der erste Bestandteil eine Öl-in-Wasser-Emulsion ist, die DHA enthält und der amino-enthaltende Bestandteil ein aminosubstituiertes Silicon ist.

9. Zusammensetzung nach Anspruch 8, wobei das aminosubstituierte Silicon eine Verbindung der Formel (I) ist 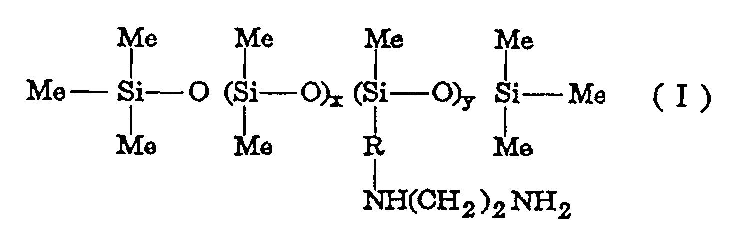 worin:
x = 48-148,
y = 3-15, und
R eine divalente Alkylengruppe mit 3-6 Kohlenstoffatomen ist.

10. Verfahren, um menschlicher Haut eine künstlich induzierte Sonnenbräune zu verleihen, das umfaßt,
Behandeln der Haut mit einer ersten Zusammensetzung, die wenigstens etwa 2% eines amino-enthaltenden Bestandteils aufweist, der ausgewählt ist aus der Gruppe, die besteht aus aminosubstituierten Siliconen und Polyaziridinen, und
Auftragen einer zweiten Zusammensetzung mit etwa 1-20% DHA auf die Haut.

11. Verfahren nach Anspruch 10, wobei die erste Zusammensetzung wenigstens eine von einer Wasser-Alkohollösung oder einer Öl-in-Wasser-Emulsion ist.

12. Verfahren nach Anspruch 11, wobei die Wasser-Alkohollösung Wasser und Ethanol ist.

13. Verfahren nach Anspruch 11, wobei DHA wenigstens etwa 1% der zweiten Zusammensetzung ausmacht.

14. Verfahren nach Anspruch 13, wobei DHA etwa 5-8% der zweiten Zusammensetzung ausmacht.

15. Verfahren nach Anspruch 10, wobei die zweite Zusammensetzung eine Öl-in-Wasser-Emulsion ist, die etwa 5-8% DHA enthält.

16. Verfahren nach Anspruch 10, wobei das Polyaziridin Polyethylenimin ist.

17. Verfahren nach Anspruch 10, wobei die erste Zusammensetzung Polyethylenimin einschließt, und die zweite Zusammensetzung eine Wasser-in-Öl-Emulsion ist, die DHA enthält.

18. Verfahren nach Anspruch 10, wobei die erste Zusammensetzung ein aminosubstituiertes Silicon der Formel (I) umfaßt 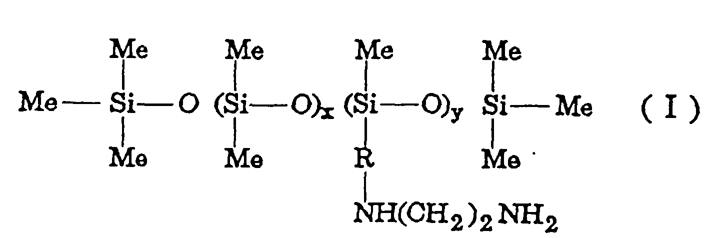 worin:
x = 48-148,
y = 3-15; und
R eine divalente Alkylengruppe mit 3-6 Kohlenstoffatomen ist;
und die zweite Zusammensetzung eine Wasser-in-Öl-Emulsion ist, die DHA enthält.

19. Verfahren nach Anspruch 10, wobei die Behandlung mit der ersten Zusammensetzung und Auftragen der Zusammensetzung, die DHA enthält, im wesentlichen gleichzeitig erfolgen.

20. Verfahren nach Anspruch 10, wobei die erste und die zweite Zusammensetzung gleichzeitig aufgetragen werden.

21. Verfahren nach Anspruch 10, wobei sich die erste und die zweite Zusammensetzung in einer Zweiphasenzusammensetzung befinden.

22. Verfahren nach Anspruch 10, um menschlicher Haut eine künstlich induzierte Sonnenbräune zu verleihen, das umfaßt:
(a) Behandeln der Haut mit einer ersten Zusammensetzung, die wenigstens etwa 2% eines amino-enthaltenden Bestandteils umfaßt, der ausgewählt ist aus der Gruppe, die besteht aus einem aminosubstituierten Silicon oder wenigstens einem Polyaziridin; und
(b) Auftragen einer zweiten Zusammensetzung mit etwa 1-20% DHA auf die Haut.

23. Verfahren nach Anspruch 22, wobei die erste und die zweite Zusammensetzung gleichzeitig aufgetragen werden.

24. Verfahren nach Anspruch 22, wobei sich die erste und die zweite Zusammensetzung in einer Zweiphasenzusammensetzung befinden.

## Revendications

1. Une composition cosmétique applicable à la peau humaine pour conférer à celle-ci un bronzage artificiel, comprenant:
- un premier composant comprenant environ 1 à 20 % de DHA ; et
- un second composant comprenant au moins 2 % d'un composant contenant une fonctionnalité amine, choisi dans le groupe consistant en silicones aminées et polyaziridines, le second composant étant disponible pour réagir avec ledit premier composant pour augmenter la vitesse et le degré de bronzage conféré à la peau humaine.

2. La composition selon la revendication 1, dans laquelle le premier composant comprend au moins un choisi parmi une solution hydroalcoolique, une émulsion aqueuse, sans huile, sans alcool, à pulvériser, ou une émulsion huile-dans-eau.

3. La composition selon la revendication 2, dans laquelle ladite solution hydroalcoolique comprend de l'eau et de l'éthanol.

4. La composition selon la revendication 2, dans laquelle ledit DHA représente environ 5 à 8 % dudit premier composant.

5. La composition selon la revendication 1, dans laquelle ledit premier composant est une émulsion huile-dans-eau contenant environ 5 à 8 % de DHA.

6. La composition selon la revendication 1, dans laquelle ladite polyaziridine est le polyéthylèneimine.

7. La composition selon la revendication 1, dans laquelle ledit premier composant est une émulsion huile-dans-eau comprenant du DHA et ledit second composant est le polyéthylèneimine.

8. La composition selon la revendication 1, dans laquelle ledit premier composant est une émulsion huile-dans-eau contenant du DHA et ledit composant contenant une fonctionnalité amine est une silicone aminée.

9. La composition selon la revendication 8, dans laquelle ladite silicone aminée est un composé de formule (I): 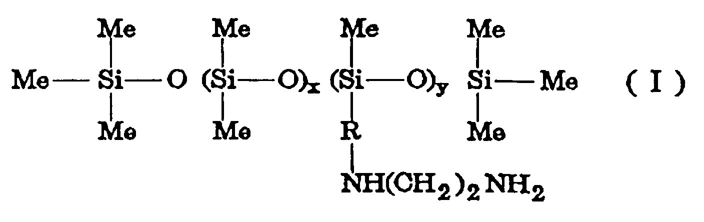 dans laquelle:
x est égal à 48 à 148,
y est égal à 3 à 15, et
R est un radical alkylène divalent comportant 3 à 6 atomes de carbone.

10. Une méthode pour conférer un bronzage provoqué artificiellement à la peau humaine, comprenant:
- le traitement de la peau à l'aide d'une première composition comportant au moins environ 2 % d'un composant contenant une fonctionnalité amine choisi dans le groupe consistant en silicones aminées et polyaziridines, et
- l'application sur la peau d'une seconde composition comportant environ 1 à 20 % de DHA.

11. La méthode selon la revendication 10, dans laquelle ladite première composition est au moins une choisie parmi une solution hydroalcoolique ou une émulsion huile-dans-eau.

12. La méthode selon la revendication il, dans laquelle ladite solution hydroalcoolique est une solution aqueuse éthanolique.

13. La méthode selon la revendication 11, dans laquelle ledit DHA représente au moins environ 1 % de ladite seconde composition.

14. La méthode selon la revendication 13, dans laquelle ledit DHA représente environ 5 à 8 % de ladite seconde composition.

15. La méthode selon la revendication 10, dans laquelle ladite seconde composition est une émulsion huile-dans-eau contenant environ 5 à 8 % de DHA.

16. La méthode selon la revendication 10, dans laquelle ladite polyaziridine est le polyéthylèneimine.

17. La méthode selon la revendication 10, dans laquelle ladite première composition comprend du polyéthylèneimine et ladite seconde composition est une émulsion eau-dans-huile contenant du DHA.

18. La méthode selon la revendication 10, dans laquelle ladite première composition comprend une silicone aminée de formule (I): 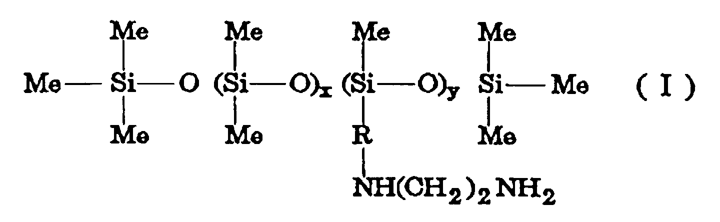 dans laquelle :
x est égal à 48 à 148,
y est égal à 3 à 15, et
R est un radical alkylène divalent comportant 3 à 6 atomes de carbone ; et ladite seconde composition est une émulsion huile-dans-eau contenant du DHA.

19. La méthode selon la revendication 10, dans laquelle ledit traitement à l'aide de ladite première composition et ladite application de ladite composition contenant du DHA sont effectués sensiblement simultanément.

20. La méthode selon la revendication 10, dans laquelle lesdites première et seconde compositions sont appliquées simultanément.

21. La méthode selon la revendication 10, dans laquelle ladite première composition et ladite seconde composition se trouvent dans une composition à deux phases.

22. Une méthode pour conférer un bronzage provoqué artificiellement à la peau humaine selon la revendication 10, comprenant :
(a) le traitement de la peau à l'aide d'une première composition comprenant au moins environ 2 % d'un composant contenant la fonctionnalité amine choisie dans le groupe consistant en une silicone aminée ou au moins une polyaziridine, et
(b) l'application sur la peau d'une seconde composition comportant environ l à 20 % de DHA.

23. La méthode selon la revendication 22, dans laquelle ladite première composition et ladite seconde composition sont appliquées simultanément.

24. La méthode selon la revendication 22, dans laquelle ladite première composition et ladite seconde composition se trouvent dans une composition à deux phases.
